# EUROPÄISCHE PATENTSCHRIFT

(11) **EP 1 289 575 B1**
(45) Veröffentlichungstag und Bekanntmachung des Hinweises auf die Patenterteilung: **28.07.2004**
(21) Anmeldenummer: 01943148.5
(22) Anmeldetag: 28.05.2001
(51) Int. Cl.: A61L 27/38, A61L 2/00

(54) **VERFAHREN ZUR HERSTELLUNG EINES EMPFÄNGERSPEZIFISCHEN GEWEBE-TRANSPLANTATES**
METHOD FOR PRODUCING A RECIPIENT-SPECIFIC TISSUE TRANSPLANT
PROCEDE DE PRODUCTION D'UN GREFFON DE TISSU SPECIFIQUE A UN RECEPTEUR

(30) Priorität: 29.05.2000 DE 10026442
(43) Veröffentlichungstag der Anmeldung: 12.03.2003
(73) Patentinhaber: Bader, Augustinus, 31275 Lehrte-Immensen (DE)
(72) Erfinder: Bader, Augustinus, 31275 Lehrte-Immensen (DE)
(74) Vertreter: Müller, Volkmar
(86) Internationale Anmeldenummer: PCT/DE2001/001985
(87) Internationale Veröffentlichungsnummer: WO 2001/091819

(56) Entgegenhaltungen:
- EP-A- 0 424 159
- US-A- 5 192 312

## Beschreibung

Die Erfindung betrifft ein Verfahren zur Herstellung eines empfängerspezifischen Gewebe - Transplantats auf der Basis einer autologenen, allogenen oder xenogenen Gewebematrix in nativer oder azellularisierter Form, d. h. einem für die Transplantation vorgesehenen biologischen Gewebe und darauf aufgebrachten empfängerverträglichen Zellen.

Das Verfahren ist allgemein anwendbar auf Verfahren zur kontrollierten Züchtung von biologischem Gewebe unter Verwendung von autologen, allogenen oder xenogenen Ausgangsmaterialien.

In der Transplantationsmedizin besteht ein großes Bedürfnis nach geeigneten, in möglichst geringem Maße adverse Reaktionen des Transplantat - Empfängers auslösenden Transplantaten. Nur in bestimmten Fällen ist es möglich, das Transplantat aus dem Körper des Empfängers selbst zu entnehmen und zu verpflanzen. Aus immunbiologischer Sicht sind diese Transplantationen am unbedenklichsten, bei bestimmten Gefäßen oder Organen sowie bei größeren zu ersetzenden Hautbereichen besteht diese Möglichkeit jedoch nicht. Für bestimmte Organe kommen heute praktisch nur allogene Transplantate fremder Spender oder - vielfach im orthopädischen Bereich - synthetische Implantate aus Kunststoffen, Metallen, Keramik usw. bzw. verschiedenen Verbundstoffen in Betracht. Bei der Verwendung allogener Materialien, wie z. B. Spenderorganen ist eine ständige für den Organismus des Empfängers belastende Immunsuppression nötig. Dennoch kommt es häufig zu Abstoßungsreaktionen als ernsten Komplikationen. Auch künstliche Materialien können zu Abstoßungsreaktionen und entzündlichen Prozessen führen, die das Operationsergebnis zunichte machen.

Aus verschiedenen Gründen wird heute oft versucht, xenogenes Material (tierischen Ursprungs) zu verwenden. Vorteilhaft ist hierbei vor allem die bessere Verfügbarkeit dieses Materials gegenüber allogenen (Spender-) Materialien. Auch ist ein solches "biologisches Material" flexibler als ein künstliches Material und passt sich an manchen Stellen dem Empfängerkörper besser an. Das xenogene Transplantationsmaterial ist jedoch deshalb problematisch, da es stark antigen ist.

Es wird daher seit längerer Zeit versucht, xenogene und allogene Transplantationsmaterialien - speziell verschiedene für eine Transplantation vorgesehene Gewebe - empfängerverträglich zu machen. Hierzu werden häufig in geeigneter Weise vorbereitete Gewebe (allogene, xenogene) mit empfängereigenen, d. h. autologen, oder empfängerverträglichen, häufig genetisch veränderten allogenen und xenogenen Zellen besiedelt. Dadurch werden für den Empfänger speziell nachgezüchtete Gewebe erhalten.

Hierbei bereitet es häufig Schwierigkeiten, dass die empfängerverträglichen Zellen auf der Grundmatrix naturähnlich wieder anwachsen. Ein weiteres Problem besteht darin, dass xenogene oder allogene Ausgangsmaterialien von Krankheitskeimen (Viren, Bakterien) befallen sein können, die für den Empfänger neu und ggf. auch fremd sind, so dass das Transplantat für den Empfänger unnötige Gefahren und Belastungen mitbringt.

Aus der US-A 5,192,312 ist ein Verfahren zur Herstellung eines Gewebetransplantats aus einer Gewebematrix, welches erst durch Bestrahlung oder durch chemische Stoffe sterilisiert und anschließend mit autologen Zellen besiedelt wird, bekannt.

Die Aufgabe der Erfindung besteht daher darin, ein Verfahren der eingangs genannten Art so auszugestalten, dass die für die Transplantation vorgesehene Gewebematrix (Gewebegrundlage) für eine Besiedlung mit empfängerspezifischen Zellen optimal vorbereitet wird.

Zur Lösung dieser Aufgabe wird das gattungsgemäße Verfahren erfindungsgemäß so weitergebildet, dass vor der Besiedlung mit den empfängerverträglichen Zellen eine Sterilisation und verbesserte Vorbereitung der Besiedlung der Gewebematrix durch eine Plasmaionisation mit H₂O₂ erfolgt.

Eine Weiterbildung der Erfindung können weitere Sterilisationsschritte hinzugenommen werden, wie beispielsweise eine Detergentienbehandlung, eine γ-Bestrahlung, eine Begasung mit Ethylenoxid oder eine enzymatische Behandlung zum Abtöten der Zellen.

Vor oder nach der Sterilisierung kann zusätzlich ein- oder mehrmals gespült werden.

Im übrigen kann das Besiedeln mit empfängerverträglichen Zellen mit den im Stand der Technik hierzu bekannten und üblichen Verfahren erfolgen, die lediglich um den erfindungsgemäßen Sterilisierungsschritt erweitert werden.

Unter empfängerspezifischen Zellen werden generell für den Empfänger autologe oder immunologisch kompatible bzw. verträgliche Zellen verstanden. Es können auch zu unterschiedlichen Besiedlungs- bzw. Behandlungszeitpunkten verschiedene Arten von Zellen aufgegeben werden, wie dies im Stand der Technik soweit bekannt ist.

Überraschenderweise wurde nun gefunden, dass eine Plasmaionisation mit H₂O₂, das unterliegende Gewebe für eine nachfolgende Besiedlung mit den empfängereigenen oder - verträglichen Zellen optimal vorbereitet. Es entstehen bei der Sterilisation praktisch keine schädlichen Nebenprodukte. Eine für den Empfänger nachteilige Kontamination des Transplantats wird verhindert. Das Anwachsen der für die Besiedlung vorgesehenen Zellen auf dem erfindungsgemäß vorbereiteten Transplantat wird erleichtert.

Als empfängerverträgliche Zellen kommen grundsätzlich alle Körperzellen, beispielsweise - je nach dem unterliegenden Substrat - auch die nachfolgend beschriebenen in Frage: Bindegewebszellen (u. a. Fibroblasten, Fibrozyten), Muskelzellen (Myozyten), Endothelzellen, Hautzellen (u. a. Keratinozyten), zu Organzellen differenzierte Zellen (Herzzellen, Nierenzellen, usw.), vorzugsweise bei strukturierten Organen mit Kollagengerüst, allgemein alle Zellen, die für den Umbau eines bestimmten, für die Implantation vorgesehenen Gewebes sinnvollerweise angeboten werden können. Geeignet sind auch die Vorläuferzellen, bevorzugt aus autologen Stammzellen des Empfängers. Zu den Stammzellen gehören die oben bereits genannten.

Vor der Behandlung mit den empfängerverträglichen Zellen wird das für die Transplantation vorgesehene autologe, allogene oder xenoge, in nativer Form vorliegende oder azellularissierte Gewebe erfindungsgemäß durch Plasmaionisation mit H₂O₂ sterilisiert.

Besondere Vorteile hat die Erfindung bei xenogenen Geweben, bei denen eine Sterilisation in jedem Fall erfolgen sollte, damit sicher ausgeschlossen wird, dass artfremde Viren und Bakterien in das frisch erzeugte bioartifizielle Transplantat mit eingetragen werden. Es hat sich gezeigt, dass die erfindungsgemäße Sterilisation mit H₂O₂ bei hervorragender Verträglichkeit für das Substrat, d. h. das zu besiedelnde Transplantat, zu besonders guten (sicheren) Ergebnissen führt.

Auch bei allogenen Ausgangsgeweben sollte eine Krankheitsübertragung sicher ausgeschlossen werden; die erfindungsgemäße Sterilisation ist auch hier besonders vorteilhaft.

Als besonders geeignet wird derzeit eine Sterilisation durch Plasmaionisation angesehen, bei der eine Gasentladung in Anwesenheit von H₂O₂ erfolgt. Hierzu wird eine wässrige Lösung von Wasserstoffperoxid in eine Sterilisationskammer injiziert und vaporisiert. Unter reduziertem Umgebungsdruck wird mit Hilfe einer Radiofrequenzenergie ein niedrig - Temperatur - Plasma appliziert. Hierdurch wird ein elektrisches Feld generiert, welches ein Plasma erzeugt. In dem Plasma - Zustand wird das Wasserstoffperoxid unter Radikalbildung gespalten. Die Radikale stellen die aktive Spezies für die Sterilisation dar.

Das erfindungsgemäße Verfahren hinterlässt keine toxischen Rückstände, da nach Abschluss der Reaktion die Radikale zu Wasser, Sauerstoff und anderen nicht toxischen Produkten abreagieren. Die Verwendung von Peroxiden entspricht auch einem in vielen Zellen vorkommenden natürlichen Prozess (z. B. in Makrophagen).

Gegebenenfalls kann der Erfolg der Sterilisierung spezifisch geprüft werden, in dem beispielsweise nach der Sterilisierung auf Anwesenheit bestimmter Viren oder Bakterien, die strikt unterbunden werden sollen, getestet wird.

Das für die Transplantation vorgesehene Gewebe kann nach seiner Präparation vor einer möglicherweise erforderlichen Sterilisierung zusätzlichen nicht - denaturierenden Verfahrensschritten, ausgesetzt werden. Ein- oder mehrmaliges Spülen vor und/oder nach der Sterilisierung wird als vorteilhaft angesehen.

Nach der Besiedlung kann das Transplantat in üblicher Weise weiterbehandelt oder sofort zum Operationsort überführt werden.

### Vergleichsbeispiel 1:

### Vorbereitung von Kollagenstrukturen tierischen Ursprungs für Implantationen

Eine Kollagenmatrix wird in einer wässrigen Lösung bei pH 3,8 in einem Behältnis aus Glas bisher üblicherweise mit chemischen Reagenzien wie Chloroform sterilisiert. Dieses verdampft unter kontinuierlichem Rühren bei 4 °C über einen Zeitraum von 48 - 72 Stunden. Danach muss bis zu 2 Wochen gewartet werden, um Restbestände, die noch cytotoxisch wirken, abzudampfen.

Im Sinne der Erfindung wird die Kollagenlösung mit H₂O₂ in stark verdünnter Form (1 % oder 0,1 %) entsprechend unter kontinuierlichem Rühren bei 4 °C behandelt. Ein Vorteil ist die raschere Einsetzbarkeit nach bereits 48 Stunden. Ebenso kann die Lösung durch Plasmaionisation behandelt werden. Dieser Prozess wird in einem zu diesem Zwecke gaszugänglichen Behältnis durchgeführt, wobei die Schichtdicke des Mediums im Idealfall bis zu 3 cm betragen kann. Der Plasmaionisationsprozess führt hierbei zu einer beginnenden Quervernetzung des Kollagens und damit zu einer Formstabilisierung. Die nachfolgende Besiedlung dieser Strukturen mit porkinen und humanen Fibroblasten und Leberzellen hat sich als besonders erfolgreich herausgestellt, da Adhäsionsprozesse und auch Migrationsprozesse innerhalb der Matrix erleichtert sind.

### Vergleichsbeispiel 2

### Vorbereitung von Kollagenstrukturen tierischen Ursprungs für Implantationen

Haut oder Herzklappenstrukturen aus nativer Matrix werden mit einer 0,1 %tigen H₂O₂ Lösung behandelt. Der Behandlungsprozess erfolgt durch Immersion in die H₂O₂ Lösung für 15 Minuten und nachfolgendem Waschen mit steriler Kochsalzlösung. Der Prozess kann mehrfach wiederholt werden. Die Folge ist eine Desinfektion mit einhergehender Verbesserung der Besiedelbarkeit der Implantatstrukturen.

### Vergleichsbeispiel 3:

### Sterilisation strukturierter Kollagenmatrices in Bioreaktoren

Bioreaktoren aus Kunststoffen, die oder mit Kunststoffteilen enthalten, werden mit den Inhaltsmaterialien zum Beispiel Kollagenfliese als komplette Einheit sterilisiert. Hierzu eignen sich sogenannte Flachmembranbioreaktoren (FMB) oder Rotationsbioreaktoren. Die Flachmembranbioreaktoren werden zum Beispiel mit Kollagenlösung beschichtet, indem ein Teil einer 1,1 %tigen Kollagenlösung mit einem 10.fach Konzentrat von DMEM (Dulbeccos Modified Eagle Medium) eingebracht und verteilt werden. Danach werden die Bioreaktoren in der Ionisationskammer mit H₂O₂ behandelt. Dieser Sterilisationsprozess führt zu einer Verbesserung der Stabilität der Beschichtung, einer höheren internen Stabilität und einer besseren Besiedelbarkeit der Implantate.

### Beispiel 1:

### Sterilisation von Gewebestücken aus nativer Matrix in Bioreaktoren

Entsprechend dem Vergleichsbeispiel 2 können die zur Implantation vorgesehenen Strukturen auch nach Fixation oder Einbringung in Bioreaktoren behandelt werden. Hautgewebestücke oder zum Beispiel kardiovaskuläre Strukturen wie Gefäße, oder Patches werden in Bioreaktoren fixiert und anschließend ohne weitere Behandlung in die Plasmaionisationskammer gebracht. Dort werden diese zyklisch mit H₂O₂ Plasma behandelt und danach direkt ohne weitere Behandlung in diesen Bioreaktoren rebesiedelt. Hierzu werden Isolations- und Besiedlungsprotokolle verwendet.

## Patentansprüche

1. Verfahren zur Herstellung eines empfängerspezifischen Gewebe-Transplantats auf der Basis einer autologenen, allogenen oder xenogenen Gewebematrix in nativer oder azellularisierter Form und darauf angesiedelten empfängerverträglichen Zellen, **dadurch gekennzeichnet, dass** vor der Besiedlung mit den empfängerverträglichen Zellen eine Sterilisation und Verbesserung der Besiedlungsfähigkeit der Gewebematrix durch eine Plasmaionisation mit H₂O₂ erfolgt.

2. Verfahren nach Anspruch 1, **dadurch gekennzeichnet, dass** zusätzlich eine weitere Sterilisation, vorzugsweise mit H₂O₂, eine Detergentienbehandlung, eine γ-Bestrahlung, eine Begasung mit Ethylenoxid oder eine enzymatische Behandlung durchgeführt wird.

3. Verfahren nach einem der Ansprüche 1 bis 2, **dadurch gekennzeichnet, dass** das für die Transplantation vorgesehene Gewebe nach seiner Präparation, vorzugsweise vor oder nach der Sterilisierung, zusätzlich ein oder mehrmals gespült wird.

## Claims

1. Process for manufacturing a receiver-specific tissue transplant on the basis of an autologous, allogenic or xenogenic tissue matrix in a native or acellularised form and of receiver-compatible cells introduced to it, which is **characterized by** sterilizing and improving the tissue matrix's capacity for population by applying plasma ionisation with H₂O₂ before settling receiver-compatible cells on it.

2. Process according to Claim 1, which is **characterized by** carrying out another sterilisation process, preferably with H₂O₂, a treatment with detergents, gamma radiation, gas exposure with ethylene oxide or an enzymatic treatment.

3. Process according to either Claim 1 or 2, which is **characterized by** the tissue provided for transplantation being rinsed after its preparation once or several times preferably before or after sterilisation.

## Revendications

1. Procédure de fabrication d'un transplant de tissu suivant les spécifications du récepteur sur la base d'une matrice de tissu autologue, allogènique ou xénogène en forme native ou acellula risée et de cellules introduites sur ce transplant et qui sont compatibles au récepteur, caractéisée en ce qu'il se réalise, avant l'introduction des cellules compatibles au récepteur, une stérilisation et amélioration de la capacité de recevoir des de la matrice de tissu à l'intermédiaire d'une ionisation à plasma avec H₂O₂.

2. Procédure selon la revendication 1, **caractérisée en ce qu'**il se réalise aussi une stérilisation supplémentaire, de préférence avec H₂O₂, un traitement par détergents, une irradiation aux rayons gamma, un gazage avec de l'oxyde d'éthylène ou un traitement enzymatique.

3. Procédure selon l'une des revendications 1 ou 2, **caractérisée en ce que** le tissu prévu pour la transplantation est lavé, de préférence avant ou après la stérilisation, encore une ou plusieurs fois après sa préparation.
